# EUROPEAN PATENT SPECIFICATION

(11) **EP 2 213 660 B1**
(45) Date of publication and mention of the grant of the patent: **12.12.2012**
(21) Application number: 08845963.1
(22) Date of filing: 01.10.2008
(51) Int. Cl.: C07D 209/42, C07D 401/12, C07D 403/12, C07D 413/12, A61K 31/404, C07D 401/06, C07D 403/06, C07D 413/06

(54) **5-SUBSTITUTED INDOL-3-CARBOXYLIC ACID DERIVATIVES EXHIBITING ANTIVIRAL ACTIVITY A METHOD FOR THE PRODUCTION AND USE THEREOF**
5-SUBSTITUIERTE INDOL-3-CARBONSÄUREDERIVATE MIT ANTIVIRALER WIRKUNG SOWIE VERFAHREN ZU IHRER HERSTELLUNG UND VERWENDUNG
DÉRIVÉS 5-SUBSTITUÉS DE L'ACIDE INDOL-3-CARBONIQUE POSSÉDANT UNE ACTIVITÉ ANTIVIRALE, PROCÉDÉ DE FABRICATION ET D'UTILISATION

(30) Priority: 31.10.2007 RU 2007140220
(43) Date of publication of application: 04.08.2010
(73) Proprietor: Research and development company Medbiopharm Ltd., 249030 Obninsk (RU)
(72) Inventor: VERKHOVSKY, Jury Grigorievich, Kaluzhskaya obl. 249032 (RU); TSYSHKOVA, Nina Gavrilovna, Kaluzhskaya obl. 249032 (RU); ROZIEV, Rakhimdzhan Akhmetdzhanovich, Kaluzhskaya obl. 249031 (RU); TSYB, Anatoliy Fedorovich, Kaluzhskaya obl. 249039 (RU); GONCHAROVA, Anna Yakovlevna, Kaluzhskaya obl. 249031 (RU); TROFIMOV, Fedor Alexandrovich, Kaluzhskaya obl. 249034 (RU)
(74) Representative: von Füner, Nicolai
(86) International application number: PCT/RU2008/000629
(87) International publication number: WO 2009/058051

(56) References cited:
- WO-A2-2007/120079
- TROFIMOV F A ET AL: "Studies in the quinone field XLVI. Synthesis of thioethers and sulfones in the 5-hydroxyindole series" PHARMACEUTICAL CHEMISTRY JOURNAL, SPRINGER NEW YORK LLC, US LNKD- DOI:10.1007/BF00759819, vol. 3, no. 11, 1 November 1969 (1969-11-01), pages 641-644, XP008128297 ISSN: 0091-150X
- SHVEDOV V I ET AL: "Investigation of quinones" PHARMACEUTICAL CHEMISTRY JOURNAL, SPRINGER NEW YORK LLC, US LNKD- DOI:10.1007/BF00771345, vol. 4, no. 9, 1 September 1970 (1970-09-01), pages 473-477, XP008128298 ISSN: 0091-150X
- SHVEDOV V I ET AL: "Synthesis of aminoalkyl derivatives of 2-benzyl-5-hydroxyindole" CHEMISTRY OF HETEROCYCLIC COMPOUNDS (A TRANSLATION OF KHIMIYAGETEROTSIKLICHESKIKH SOEDINENII), PLENUM PRESS CO., NEW YORK, NY, US LNKD- DOI:10.1007/BF00473085, vol. 7, no. 2, 1 February 1971 (1971-02-01), pages 190-194, XP008128299 ISSN: 0009-3122
- DATABASE ACS 10 August 2006 XP008138178 Retrieved from STN Database accession no. RN 900365-56-2
- DATABASE ACS 10 August 2006 XP008138173 Retrieved from STN Database accession no. RN 900365-53-9
- DATABASE ACS 10 August 2006 XP008138174 Retrieved from STN Database accession no. RN 900365-50-6
- DATABASE ACS 27 June 2006 XP008138175 Retrieved from STN Database accession no. RN 889634-13-3
- DATABASE ACS 10 March 2005 XP008138176 Retrieved from STN Database accession no. RN 844882-49-1
- DATABASE ACS 18 August 2004 XP008138169 Retrieved from STN Database accession no. RN 728036-88-2
- DATABASE ACS 18 August 2004 XP008138167 Retrieved from STN Database accession no. RN 728034-42-2
- DATABASE ACS 17 August 2004 XP008138170 Retrieved from STN Database accession no. RN 727685-92-9
- DATABASE ACS 17 August 2004 XP008138172 Retrieved from STN Database accession no. RN 727681-37-0
- DATABASE ACS 17 August 2004 XP008138202 Retrieved from STN Database accession no. RN 727681-35-8
- DATABASE ACS 17 August 2004 XP008138201 Retrieved from STN Database accession no. RN 727681-34-7
- DATABASE ACS 17 August 2004 XP008138166 Retrieved from STN Database accession no. RN 727679-37-0
- DATABASE ACS 17 August 2004 XP008138168 Retrieved from STN Database accession no. RN 727662-08-0
- DATABASE ACS 16 August 2004 XP008138203 Retrieved from STN Database accession no. RN 727420-39-5
- DATABASE ACS 06 July 2004 XP008138171 Retrieved from STN Database accession no. RN 704871-85-2
- DATABASE ACS 04 March 2007 XP008138196 Retrieved from STN Database accession no. RN 924714-05-6 & DATABASE ACS 17 July 2006 STN Database accession no. RN 893770-74-6
- DATABASE ACS 27 June 2006 XP008138198 Retrieved from STN Database accession no. RN 889633-17-4
- DATABASE ACS 17 August 2004 XP008138200 Retrieved from STN Database accession no. RN 727681-40-5
- DATABASE ACS 28 March 2001 XP008138206 Retrieved from STN Database accession no. RN 329209-21-4
- DATABASE ACS 02 January 2001 XP008138195 Retrieved from STN Database accession no. RN 312513-24-9
- 'Khimiko-pharmatsevtichesky zhurnal.' MEDITSINE vol. 3, no. 11, 1969, pages 25 - 29
- GADAGINAMATH G. S. ET AL.: 'Synthesis and antibacterial activity of novel 1-butyl- 2-phenoxy/2-phenylthio/2-aminomethyl-5-meth oxyindole derivatives.' POLISH J. CHEM. vol. 71, 1997, pages 923 - 928, XP009115934
- 'Khimicheskaya entsiklopediya.', vol. 1, 1998, NAUCHNOE IZDATELSTVO, MOSCOW article I.L.KNUNYANTS.: 'Bolshaya Possiiskaya entsiklopediya', page 127

## Description

The invention relates to the novel 5-hydmxymdol-3-carboxylic acid derivatives having antiviral action, which can be used for prophylaxis and treatment of such widespread viral disease as influenza.

As a rule, the existing antiviral preparations are active only in relation to certain viruses. At the present time more than thousand types of viruses are already discovered and about half of them represent danger for human. Therefore the creation of the novel antiviral preparations is very necessary. Mechanism of action of the modem antiviral preparations consists in the blocking of one of the reproduction stage of viruses in the cells of virus carrier (human), which include stage of virus attachment to the cell and penetration inside it, incorporation of the virus nucleic acid in host cell genome, synthesis of inherent DNA and RNA, synthesis and assembly of the inherent virus proteins. Most of the existing antiviral preparations are the nucleoside analogues. They are effective, but after the use thereof for a number of patients relapses are observed, the ricochet phenomenon leading to disease exacerbation is possible. In addition, for a number of antiviral preparations the development of the virus stability against them is typical. All of the recited factors testify that only creation of novel antiviral preparations can ensure the progress in the treatment of viral diseases. There are many publications which describe 6-halogen-5-hydroxyindol-3-carboxylate derivatives.

The most well-known and effective compound having antiviral action is Arbidol (1-methyl-2-phenylthiomethyl-3-carbethoxy-4-dimethylaminomethyl-5-hydroxy-6-bromindol) (see, for example, patent RU 2033156). Arbidol can stimulate various immune functions, it is well tolerated at use thereof for prophylaxis and treatment of viral infections. But arbidol is not effective enough to some virus strains, for example, to influenza A and B virus strains.

In the article «Synthesis and pharmacological study of 1,2,3,4-tetrahydropyrazino[1,2-a]indols», Chemico-pharmaceutical journal, 1992, 26(9-10), Tsyshkova N.G. et al. describe compounds of the general formula (3) 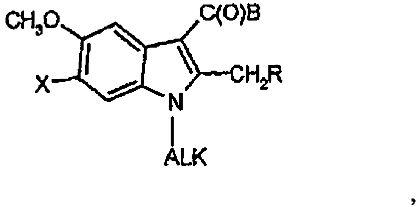 where X-H, Br, B- OEt, R-H, Alk-CH₂CH₂Br are intermediate products for the preparation of biologically active compounds.

Synthesis and antibacterial activity of the compounds of the general formula (3), where **X-**Br, **R**-OR,SR¹,R² (R-Ph, 2(4)Cl-Ph, β-naphthyl and others, R¹-Ph, 4-ClPh, R²-NMePh, 4-Br-PhNH and others), **B**-OEt, **Alk**-n-butyl, are described by Gadaginamatsh. G.S. et al. in Polish Journal of chemistry, 1997, 71(7)923-28.

W02006/42045A1 discloses synthesis and features of 6-F-indolcarboxylates as chemokine CCR-5 receptor antagonists.

WO2007/014851A2 discloses N-(C₂₋₆-alkyl)indol-3-ylcarbonylpiperazine derivatives and 3-carbonylpiperidine derivatives, which are optionally substituted in the second position of indol by alkylcarbonyl-, aminoalkylcarbonyl- or alkyl group, optionally substituted by halogen atoms, amino, alkylcarbonyloxy, alkyl-, aryl- or heteroarylcarbonylamino group. Known compounds can be used for preparation of the medicaments at treatment of various diseases, for example, at treatment of depressions, hypertension, etc.

UA 78317C2 discloses unsubstituted in the second position 1-[(indol-3-yl)carbonyl ]piperazine derivatives, which can be used as analgetic, and also the pharmaceutical compositions and the process of preparation of the compounds.

JP 06-199784, publ. 19.07.1994a, describes arylethylamines of the formula I:

Ar' -CH₂CH₂-N-(R₂)-R₁,

where Ar' - the group of the formula II 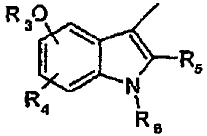 R₃- H, OH, halogen, C₁₋₆alkyl, aryl, R₄ -H, OH, halogen, C₁₋₆alkoxy, R₅ -H, halogen, C₁₋₆alkyl, R₆ -H or C₁₋₆alkyl, R₁ - the group of the formula III -COR₇, where R₇- for example, trifluormethyl, R₂-H, C₁₋₆alkyl. In particular such compound as N-[2-(5-methoxyindol-3-yl)ethyl-cyclopropyl-carboxamide, preparation and pharmaceutical compositions thereof are disclosed. Specified compounds are low-toxic, have an excellent selective compatibility with the serotonin receptor and can be used as a depressant and antianxiety, as well as antipsychotic agents.

WO2007/120079 discloses 4-AMINOMETHYL-6-BROMINE-5-HYDROXYINDOL-3-CARBOXILATE DERIVATIVES, the methods for producing said derivatives and the use thereof and the salts thereof for preparing medicinal agents for treating influenza of type A or B.

The object of the present invention is to provide novel 5-substituted indol-3-carboxylic acid derivatives having high antiviral activity and low toxicity. Moreover compounds of the invention have virus-specific activity, comparable with arbidol activity and are better than activity of some well-known compounds with antiviral action. According to the present invention compounds of the general formula (I) are proposed 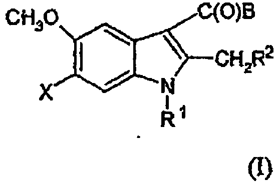 where B-is a group -N(R)₂
each R is independently selected from C₁₋₄alkyl and can be identical or different, or both groups R together with a nitrogen atom to which they are bonded, form a 5-6-membered heterocyclic ring containing 1-2 heteroatoms selected from nitrogen, oxygen and sulphur, such as pyrrolidine, piperidine, piperazine, morpholine. At which each of above-mentioned heterocyclic rings can be substituted by C₁₋₄alkyl, phenyl, benzyl, phenetyl, carbonylamino,
-COOC₁₋₄ alkyl group or -COOC₁₋₄ alkyl group and phenyl. Wherein the phenyl ring in the above-mentioned groups can have substituents selected from halogen, C₁₋₄alkyl, C₁₋₄alkoxy, and the alkyl can be linear or branched; R¹ is C₁₋₄alkyl, phenyl optionally substituted by C₁₋₄alkyl or C₁₋₄alkoxy, halogen atoms, naphthyl;
R² is C₁₋₄alkyl, -S-phenyl, -S-benzyl, -O-phenyl, wherein in each of the above-mentioned groups the phenyl ring is optionally substituted by C₁₋₄alkyl,
C₁₋₄alkoxy, halogen atoms, or
R² is an -N(R)₂ group; in which each R is independently selected from C₁₋₄alkyl and can be identical or different,
or both groups R together with a nitrogen atom to which they are bonded, form a 5-6-membered nitrogen-containing heterocyclic ring having the above mentioned meaning for the N(R)₂ group;
X is hydrogen or a halogen atom selected from Br, Cl, I, and pharmaceutically acceptable salts thereof.

Compounds of the formula (I) was not prepared before and antiviral activity of the claimed compounds of the formula (I) is unknown.

Also the invention relates to the process for preparation of the compounds of the formula (I). Compounds of the formula (I) can be prepared according to the schemes stated below: 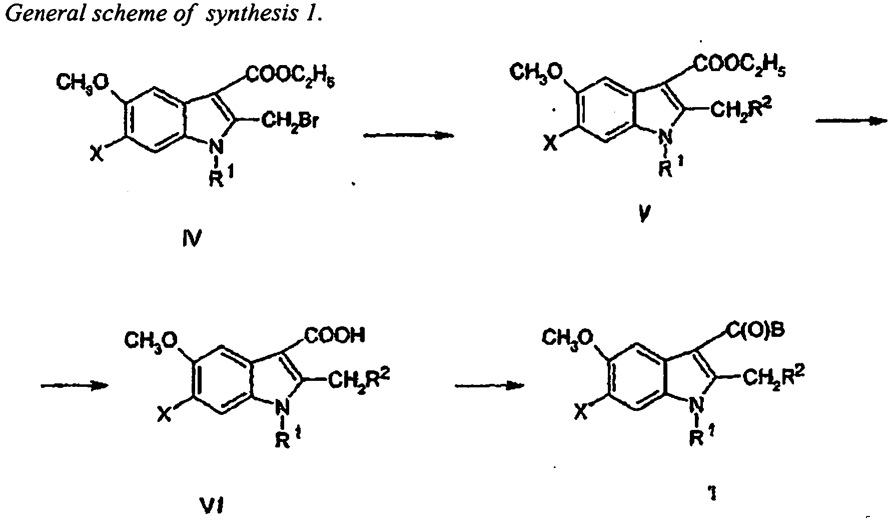 X, B, R', R² are as defined above for compounds (I).

According to the general scheme 1 the lower alkyl ester of the corresponding N-substituted 2-bromomethyl-5-methoxy-6-haloidindol-3-ylcarboxylic acid is reacted with the corresponding nucleophilic reagent, selected from the corresponding thiophenol, phenol, secondary amine, then an ester group is saponified for the preparation of the corresponding intermediately forming indol-3-ylcarboxylic acid. The last one is reacted with the corresponding secondary amine immediately or after transformation in the corresponding halogenide. At use of the immediately intermediately forming acid with amine in the reaction the dehydrating agent can be added. The amide of the formula I obtained thereby is separated or, if necessary, transformed to salt. Salts can be prepared by standard methods, for example, treatment of the compound of the formula I with the corresponding acid.

For example, hydro chlorides, mesylates, oxalates, sulphates and others refer to the salts. More particularly the process is realized as follows.

According to the scheme 2 stated below the compounds (1) and (2) can be prepared,where R² is - S-phenyl, -S-benzyl, -o-phenyl, -NR³R⁴, wherein in each of the above-mentioned groups the phenyl ring is optionally substituted by C₁₋₄alkyl, C₁₋₄alkoxy, halogen atoms.

X, N(R)₂ and R¹, R³, R⁴ are as defined above.

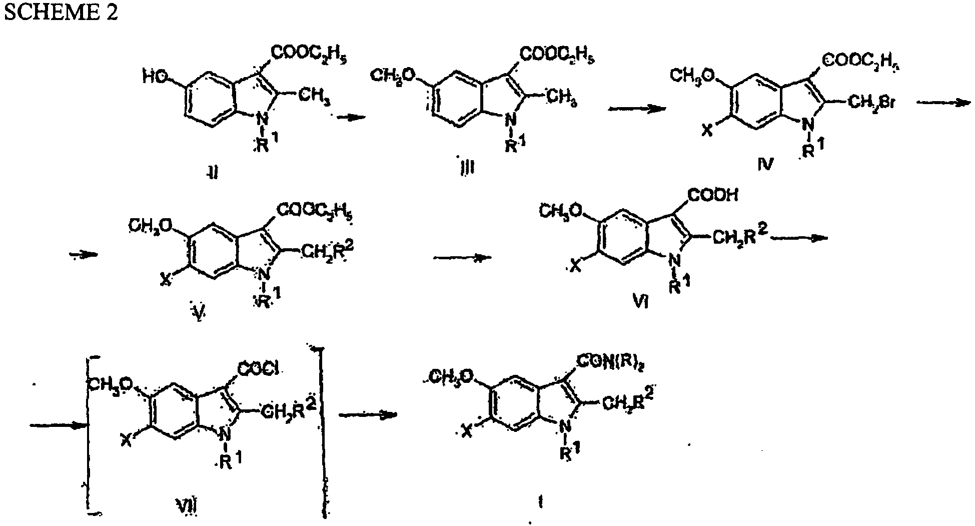

The process according to scheme 2 is realized on the basis of ethyl ester of the corresponding N-substituted 2-methyl-5-hydroxyindol-3-ylcarboxylic acid (II) by hydroxy group methylation, with the following sequential bromination of the prepared product, reaction with the nucleophilic reagent, selected from the compounds which allow to prepare the corresponding compounds of the formula I with R² as defined above.

As a parent compound 6-bromo-2-bromomethyl-1-methyl-5-ethoxyindolyl-3-carboxylic acid ethyl ester, described in the literature (HGS, 1973, Nº3. pages 308-311) can be used, which is saponified after the reaction with the corresponding nucleophilic reagents (amines, phenols, thiophenols). Obtained acids, reacting with thionyl chloride, and then with the secondary amines give the corresponding amides.

Synthesis according to the scheme 2. Parent compound according to the example 1 - 6-bromo-1-methyl-5-methoxy-2-phenylthiomethylindol-3-carboxylic acid ethyl ester (IV) is prepared according to the process with the use of the stages described in the scheme 2 and disclosed by F.A.Trofimov et al. in HGSL 973, Nº3, 308-311.

### Example 1

### 6-Bromo-1-methyl-5-methoxy-2-phenylthiomethylindol-3- carboxylic acid (VI).

3,8 g (0,009 mole) of 6-bromo-1-methyl-5-methoxy-2-phenylthiomethyl-indol-3-carboxylic acid ethyl ester, 5 g of sodium hydrate, 3 ml of water solution in 100 ml of ethyl alcohol is boiled over 3 hours. It is partially evaporated in vacuum, the water is added to the salt dissolution, and acidified with the concentrated chlorohydric acid while cooling. Precipitate is filtered off, washed with the water. Yield is 3,4 g (92%). Melting temperature is 213 °C (decomposition, from dioxane).
Found, %C : 52,97; H 3,94. C₁₈H₁₆BrNO₃S. Calculated, %: C 53,91; H 3,96.

Analogously 6-bromo-5-methoxy-1-phenyl-2-phenylthiomethyl-indol-3-carboxylic acid is obtained, melting temperature is 200-202°C (from dioxane), found, %: C 59,03, H 4,22, C₂₃H₂₈BrNO₃S. Calculated, %: C58,96; H 3,88.

### Example 2

6-Bromo-1-methyl-5-methoxy-2-phenylthiomethylindol-3-carboxylic acid chloride (VII). At room temperature 2 ml of thionyl chloride and 1 drop of dimethylformamide are added to suspension of 2,03 g (0,005 mole) of compound VI in 20 ml of dioxane. Then heated to dissolution on the water bath and left for a day at room temperature. Dioxane and thionyl chloride excess are distilled off in a vacuum, hexane is added to the residue. Precipitated 6-bromo-1-methyl-5-methoxy-2- phenylthiomethylindol-3-carboxylic acid chloride (VII) is filtered off, washed with hexane and used in the next stage without purification.

### Example 3

### 1-{[6-Bromo-1-methyl-5-methoxy-2-phenylthiomethyl-1-H-indol-3-yl]carbonyl}-4-benzylpiperazine (A)

0,54 g (0,00306 mole) of 4-benzylpiperazine in 5 ml of benzole and 0,45 ml of triethylamine are added to 1,3g (0,00306 mole) of VII solution in 10 ml of benzole. Then left for a day at room temperature. Benzole is distilled in vacuum; water is added to the oily residue, and then decanted from the oily residue. Then ethanol is added to residue, the mixture is cooled, precipitate is filtered off. Yield is 1,35g (78,4%), melting temperature is 167-169°C (from acetone).

Hydrochloride is obtained at concentrated hydrochloric acid addition to the base solution in acetone, melting temperature is 220°C (from aqueous alcohol).

Found, %: C 58,71; H 5,24; N 6,84; S 5,28, C₂₉H₃₁BrClN₃O₂S. Calculated, %: C 57,96; H5,20; N 6,99; S 5,33.

Analogously obtained:
1-{[6-Bromo-1-methyl-5-methoxy-2-phenylthiomethyl-1-H-indol-3-yl] carbonyl}pyrrolidine (B), melting temperature is 150°C (from alcohol)
1-{[6-Bromo-1-methyl-5-methoxy-2-phenylthiomethyl-1H-indol-3-yl] carbonyl}piperidine(C), melting temperature is 165 °C (from alcohol with acetone)
1-{[6-Bromo-1-methyl-5-methoxy-2-phenylthiomethyl-1-H-indol-3 -yl] carbonyl}morpholine, melting temperature is 147°C (from alcohol)
1-{[6-Bromo-1-methyl-5-methoxy-2-phenylthiomethyl-1-H-indol-3-yl]carbonyl}-4-methylpiperazine, melting temperature is 156°C (from aqueous alcohol)
1-{[6-Bromo-1-methyl-5-methoxy-2-phenylthiomethyl-1-H-indol-3-yl]carbonyl}-4-phenylpiperazine, hydrochloride, melting temperature is 120°C (from iso-propyl alcohol)
1-{[6-Iodine-1-methyl-5-methoxy-2-phenylthiomethyl-1-H-indol-3-yl]carbonyl}-4-phenylpiperazine, melting temperature is 163°C (from alcohol)
1-{[6-bromo-2-methyl-5-methoxy-1-phenyl-1-H-indol-3-yl]carbonyl}-4-phenyl-4-ethoxycarbonylpiperidine, melting temperature is 128°C (from alcohol)
1-{[2-methyl-5-methoxy-1-phenyl-1-H-indol-3-yl]carbonyl}-4-phenyl-4-ethoxycarbonylpiperidine, melting temperature is 140°C (from alcohol)
1-{[2-methyl-5-methoxy-1-phenyl-1-(4¹-methoxyphenyl)-1H-indol-3-yl]carbonyl}-4-phenyl-4-carbethoxypiperidine, melting temperature is 130°C (from alcohol).

### Synthesis realization according to the scheme 3

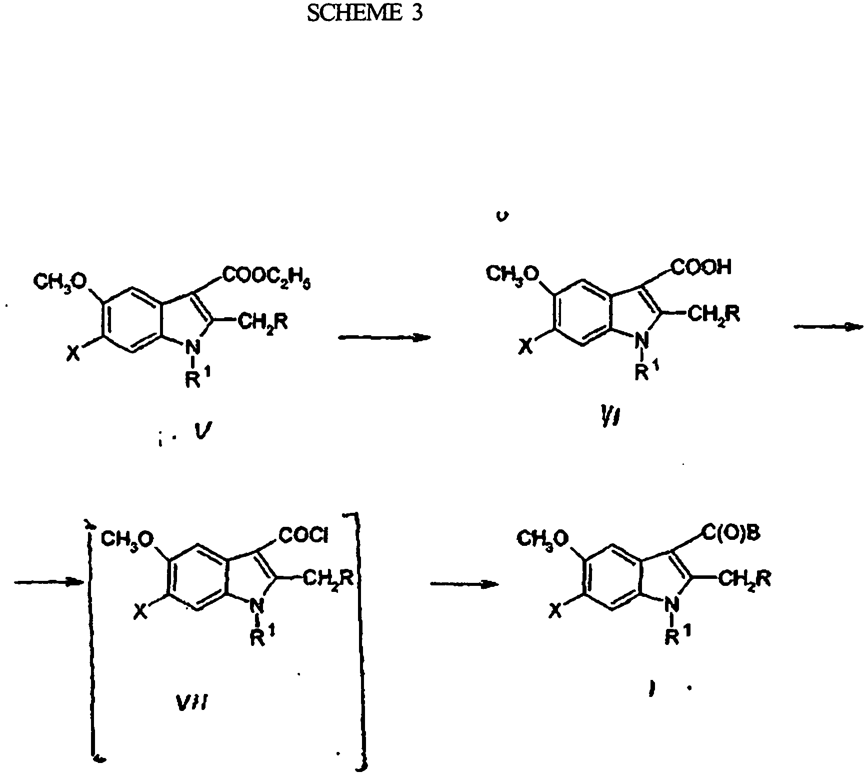

### Example 4

### 6-Bromo-1-methyl-5-methoxy-2-piperidinomethylindolyl-3-carboxylic acid hydrochloride (X).

Solution of 4,1 g (0,01 mole) of 6-bromo-1-methyl-5-methoxy-2-piperidinomethylindolyl-3-carboxylic acid ethyl ester, 6,0 g of sodium hydrate, 3 ml of water and 60 ml of ethyl alcohol is boiled during 3 hours, then 10 ml of water are added, mixture is acidified with the concentrated hydrochloric acid to the acid reaction while cooling. Precipitate is filtered off, recrystallized from aqueous alcohol. Yield is 4,1 g (98%), melting temperature is 236-238°C (from aqueous alcohol).

### Example 5

### 1-{[6-Bromo-1-mehyl-5-mehoxy-2-piperidinomethyl-1-H-indol-3-yl]cabonyl}-piperidine (XII).

Mixture of 1,25 g (0,003 mole) of compound X, 2 ml of thionyl chloride, 1 ml of dimethylformamide in 20 ml of dioxane is heated on the water bath for 3 hours. Then it is evaporated to at decreased preasure, hydrochloride acid chloride XI precipitate washed with ether. To the obtained precipitate 15 ml of benzole and mixture of 0,51 g (0,006 mole) of piperidine, 0,8 ml of triethylamine in 5 ml of benzole are added. Reaction mass is heated on the water bath for 2 hours, hydrochloride triethylamine precipitate is filtered off, washed with the hot benzole. Benzol solution is evaporated in vacuum, and hexane is added. Precipitated product is filtered off, washed with hexane. Melting temperature is 156-157°C (from alcohol and hexane mixture).
Yield is 0,7 g (52%).
Analogously 1-{[6-bromo-1-mehyl-5-memoxy-2-morpholinomehyl-1H-indol-3-yl]-carbonyl}morpholine (XII) is obtained, melting temperature is 155°C (from alcohol with hexane).
Realization of synthesis according to the scheme 2 with the use of the corresponding hydroxycompounds (for example, 4- chlorophenol, 4-hydroxypiperidine and others) as nucleophilic reagent.

### Example 6

### 6-Bromo-1-methyl-5-methoxy-2-(4'-chlorophenyloxymethyl)indolyl-3-carboxylic acid ethyl ester (XIII).

Mixture of 2,0 g (0,005 mole) of 6-bromo-2-bromomethyl-1-methyl-5-methoxyindolyl-3-carboxylic acid ethyl ester, 0,13 g (0,01 mole) of 4-chlorophenol, 3,5 g of anhydrous
potassium carbonate in 30 ml of acetone is boiled for 7 hours. Mixture is cooled, precipitate is filtered off, washed thoroughly with water and alcohol. Yield is 1,85 g (81,8%), melting temperature is 159-160°C (from acetone).

### Example 7

### 6-Bromo-1-methyl-5-methoxy-2-(4'-chlorophenyloxymethyl)indolyl-3- carboxylic acid (XV).

0,9 g (0,002 mole) of compound XIII, 1,2 g of sodium hydrate, 0,6 ml of water solution in 10 ml of ethyl alcohol and 5 ml of acetone is boiled for 2 hours. Then 10 ml of water are added and it is acidified with the concentrated hydrochloric acid while cooling.

Precipitate is filtered off, washed with water. Yield is 0,78 g (91,7%). Melting temperature is over 270°C (decomposition).

Analogously 6-bromo-1-methyl-5-methoxy-2(4¹-methoxyphenyloxymethyl)indolyl-3-carboxylic acid is obtained, melting temperature is 220°C (from dioxane)

### Example 8

### 1-{[6-Bromo-1-methyl-5-methoxy-2-(4'-chlorophenyloxy)-methyl-1-H-indol-3-yl] carbonyl}-4'-methylpiperazine

1 ml of thionyl chloride is added to 0,63 g (0,0015 mole) of compound XV solution in 10 ml of dioxane. Dioxane is distilled off from the obtained acid chloride, thionyl chloride excess, heptane is added to the residue, and precipitate is filtered off and dissolved in 10 ml of benzole. 0,3 g (0,003 mole) of 4-methylpiperazine are added to solution and mixture is left for a day at room temperature. Mixture is evaporated and water is added to the residue precipitate is filtered off and washed with water. Yield is 0,5 g (65,7%). Melting temperature is 158-160°C (from ethylacetate and petroleum-ether mixture).
Analogously obtained:
1-{[6-Bromo-1-methyl-5-methoxy-2-(4'-methoxyphenyloxymethyl)-1H-indol-3 -yl] carbonyl}-4'-benzylpiperazine, melting temperature is 155 °C (from alcohol)
1-{[6-Bromo-1-methyl-5-methoxy-2-(4'-metroxyphenyloxymethyl)-1H-indol-3-yl] carbonyl}-4'-ethoxycarbonylpiperazine, melting temperature is 163 °C (from iso-PrOH)
1-{[6-Bromo-1-methyl-5-methoxy-2-(4'-methoxyphenyloxymethyl)-1H-indol-3-yl] carbonyl}-4'-morpholine, melting temperature is 149°C (from alcohol)
1-{[6-Bromo-1-methyl-5-methoxy-2-(4'-methoxyphenyloxymethyl)-1H-indol-3-yl] carbonyl}-4'-(2-methylphenyl)piperazine, melting temperature is 184 °C (from alcohol)
1-{[6-Chloro-1-methyl-5-methoxy-2-(4'-methoxyphenyloxymethyl)-1H-indol-3-yl] carbonyl-4'-morpholine, melting temperature is 165 °C (from alcohol)
1-{[6-Bromo-1-methyl-5-methoxy-2-(4'-methoxyphenyloxymethyl)-1H-indol-3-yl] carbonyl}-4'-phenylpiperazine, 175 °C (from alcohol)
1-{[6-Bromo-1-methyl-5-methoxy-2-(4'-methoxyphenyloxymethyl)-1H-indol-3-yl] carbonyl}-4'-methylpiperazine, melting temperature is 143 °C (from alcohol)
1-{[6-Bromo-1-methyl-5-methoxy-2-(4'-methoxyphenyloxymethyl)-1H-indol-3-yl] carbonyl}-4'-(4-methylphenyl)piperazine, melting temperature is 178 °C (from alcohol)
1-{[6-Bromo-1-methyl-5-methoxy-2-(4'-methoxyphenyloxymethyl)-1H-indol-3-yl] carbonyl}-4'-(4-aminocarbonyl)piperidine, melting temperature is 163 °C (from hexane)
1-{[6-Bromo-1-methyl-5-methoxy-2-(4'-methylphenyloxymethyl)-1H-indol-3-yl] carbonyl}-4'-phenyl-4'-ethoxycarbonylpiperidine, melting temperature is 92°C (from hexane with iso-PrOH).

Structures of the prepared compounds are confirmed by PMR spectrum data, elemental analysis, individuality - by thin-layer chromatography on plates Silufol UV254.

### Biological activity tests.

### 1. Arbidol analogue effect on the influenza virus reproduction in the cells of MDSC tissue culture.

In the prior experiments cytotoxic action of compounds was investigated and maximum permissible concentrations were determined (MPC) (table 1).

Antiviral activity tests were carried out on the reference influenza A virus strain./New Caledonia/20/99 (H1N1) under conditions of the equal plurality of viral infection. For reference substance (arbidol) and tested compounds the concentrations (µg/ml) inhibiting viral reproduction on 50%(MIC₅₀) were defined and the chemotherapeutical index (MPC and MIC₅₀ ratio) was calculate, as well as substance activity at concentration of 10 µg/ml (table 1) was studied.

From the data presented in table 1 it is evident that compounds A and B in relation to the reference influenza A virus strain (H1N1) have high specific activity, comparable with the arbidol activity.

According to the MIC₅₀ ratio and chemotherapeutical index compounds A and B were even rather better than arbidol. But in concentration 10 µg/ml compound A was less effective than arbidol, while compound B didn't differ from it. Compound C exhibited low activity.

**TABLE 1**

| Inhibition of the reference influenza A virus strain /New Caledonia/20/99 (H1N1) reproduction in the cells of MDSC tissue culture with arbidol analogues. | | | | |
|---|---|---|---|---|
| Compounds | MPC compounds, µg/ml | MIC₅₀ µg/ml | chemotherapeutical index | Activity at 10 µg/ml, % |
| Arbidol | 40 | 6,0 | 6,7 | 100,0 |
| C | 25 | 9,2 | 2,7 | 52,1 |
| B | 15 | 2,0 | 7,5 | 100,0 |
| A | 40 | 5,8 | 6,9 | 64,4 |

### 2. Effectiveness evaluation of compounds on the influenzal pneumonia model of mice.

Mice were intranasally infected with influenza A virus/Aichi/2/69 (H3N3) under ether seminarcosis. Control animals were not treated in any way, while into experimental animals arbidol or compound A in a dose of 60 mg/kg/day per os was administered 24 and 1 hour before introduction of infection, then 24 hours after introduction of infection and during the following 4 days. Effectiveness of action of compounds on the influenzal pneumonia model was evaluated according to the number of mice, which have survived after introduction of virus infection and according to increase of average duration of life. Average duration of mice life was calculated according to the formula: MSD=Σf(d-1)/n, where f is the number of mice, which have died on the day d, mice which have survived are also included in f, and in this case d is 15, n is the number of mice in a group.

**TABLE 2**

| Effectiveness of compounds on the influenzal infection model of mice (virus A/Aichi/2/69) | | | | | |
|---|---|---|---|---|---|
| Compounds | n | Survived | Death rate, % | index of death decrease rate, % | Life duration (day) |
| Control of infected | 16 | 3 | 81 | | 8,1 |
| Arbidol | 10 | 8 | 20 | 61 | 12,2 |
| A | 10 | 8 | 20 | 61 | 13,1 |

From table 2 it is evident that at chemotherapeutical action test on the influenzal pneumonia model of mice compound A is not less effective than arbidol which is well-known antiviral agent.

## Claims

1. Compounds of the general formula (I) 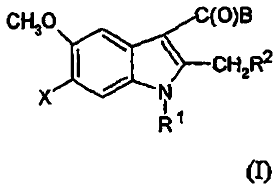 where B is a group -N(R)₂
each R is independently selected from C₁₋₄alkyl and can be identical or different, or both groups R together with a nitrogen atom to which they are bound, form a 5-6-membered heterocyclic ring containing 1-2 heteroatoms selected from nitrogen, oxygen and sulphur, such as pyrrolidine, piperidine, piperazine or morpholine, at which each of above-mentioned heterocyclic rings can be substituted by C₁₋₄alkyl, phenyl, benzyl, phenethyl, carbonylamino,
-COOC₁₋₄ alkyl group or -COOC₁₋₄ alkyl group and phenyl, which also can be substituted and have substituents selected from halogen, C₁₋₄alkyl, C₁₋₄alkoxy, and the alkyl in said groups can be linear or branched;
R¹ is C₁₋₄alkyl, phenyl, optionally substituted by C₁₋₄alkyl or C₁₋₄alkoxy, halogen atoms; R² is C₁₋₄ alkyl, -S-phenyl, -S-benzyl, -O-phenyl, wherein in each of the above-mentioned groups the phenyl ring is optionally substituted by C₁₋₄alkyl, C₁₋₄alkoxy, halogen atoms, or R² is an -N(R)₂ group;
X is hydrogen or a halogen atom selected from Br, Cl, I, and pharmaceutically acceptable salts thereof.

2. Process for the preparation of compounds of the general formula (I) 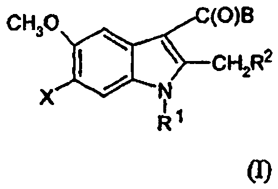 wherein B, X, R¹ are as defined in claim 1, R² is N(R)₂, which is **characterized in that** the compound of the formula (IV) 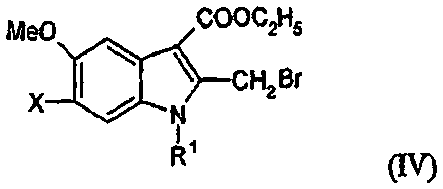 wherein X, R¹ are as defined above,
is reacted with the corresponding nucleophilic reagent, selected from corresponding phenol, thiophenol, secondary amine including fragment N(R)₂, for the preparation of the compound of the general formula (V) 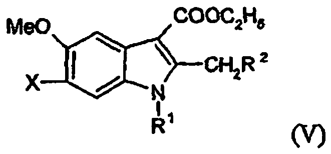 wherein X, R¹, R² are as defined above,
in which then the ester group is saponified with the preparation of the acid of the general formula (VI) 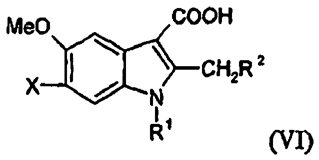 wherein X, R¹, R² are as defined above,
and the obtained acid of the formula (VI) immediately, or after transformation in the corresponding acid halide, is reacted with the corresponding secondary amine including fragment N(R)₂, with the preparation of the compound of the formula I in a free form or in the form of the pharmaceutically acceptable salt.

3. Process according to claim 2, which is **characterized in that** the compound of the general formula (IV) prepared by methylation of the compound of the formula (II), is used 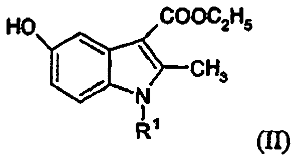 wherein X is hydrogen, R¹ is as defined above, with the preparation of the corresponding 5-methoxyindol-3-ylcarboxylic acid ester of the formula (III) 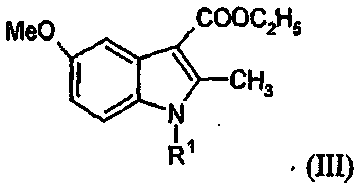 wherein R¹ is as defined above,
and with the following bromination
thereof.

4. Process according to any of claims 2 and 3, which is **characterized in that** the acid of the formula VI 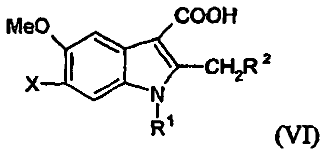 wherein R¹, R² are as defined above,
is reacted with thionyl chloride for obtainment of the corresponding acid chloride of the formula (VII) 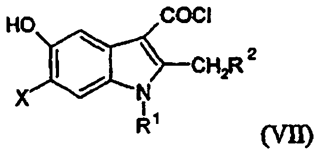 wherein R¹, R² are as defined above,
which is reacted, without separation or after separation from the reaction mass, with the secondary amine including fragment N(R)₂.

5. Process according to claim 4, which is **characterized in that** the compound of the formula VI prepared by saponification of the compound of the formula (V), is used 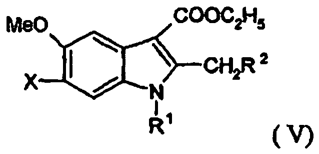 wherein X, R¹, R² are as defined above.

6. The compound of the general formula I for the preparation of a medicament for the treatment of influenza A 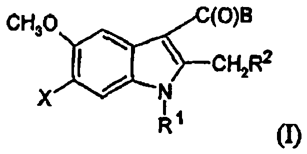 wherein X, R¹, R², B are as defined in claim 1.

## Patentansprüche

1. Verbindungen der allgemeinen Formel 1 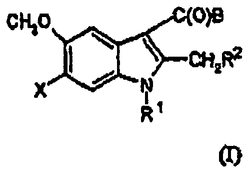 worin B eine Gruppe -N(R)₂ darstellt, wobei R jeweils unabhängig voneinander ausgewählt wird unter C₁₋₄-Alkyl und gleich oder unterschiedlich sein kann, oder beide Gruppen R zusammen mit einem Stickstoffatom, an dass sie gebunden sind, einen 5-6-gliedrigen heterocyclischen Ring mit 1 bis 2 Heteroatomen bilden, ausgewählt unter Stickstoff, Sauerstoff und Schwefel, wie Pyrrolidin, Piperidin, Piperazin oder Morpholin, wobei jeder der erwähnten heterocyclischen Ringe durch C₁₋₄-Alkyl, Phenyl, Benzyl, Phenethyl, Carbonylamino, eine -COOC₁₋₄-Alkyl- oder -COOC₁₋₄-Alkylgruppe und Phenyl substituiert sein kann, die ebenfalls substituiert sein können und Substituenten aufweisen, ausgewählt unter Halogen, C₁₋₄-Alkyl, C₁₋₄-Alkoxy, und das Alkyl in den genannten Gruppen unverzweigt oder verzweigt sein kann,
R¹ C₁₋₄-Alkyl, Phenyl, gegebenenfalls substituiert durch C₁₋₄-Alkyl oder C₁₋₄-Alkoxy, oder Halogenatome bedeutet,
R² C₁₋₄-Alkyl, -S-Phenyl, -S-Benzyl oder -O-Phenyl bedeutet, wobei jede der erwähnten Gruppen des Phenylrings gegebenenfalls substituiert ist durch C₁₋₄-Alkyl, C₁₋₄Alkoxy oder Halogenatome oder R² eine -N(R)₂-Gruppe bedeutet,
X Wasserstoff oder ein Halogenatom bedeutet, ausgewählt unter Br, Cl, I,
und pharmazeutisch verträgliche Salze davon.

2. Verfahren zur Herstellung der Verbindungen der allgemeinen Formel I 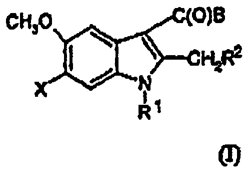 wobei B, X und R¹ wie in A.1 definiert sind und R² N(R)₂ bedeutet, **dadurch gekennzeichnet,**
**dass** die Verbindung der Formel IV 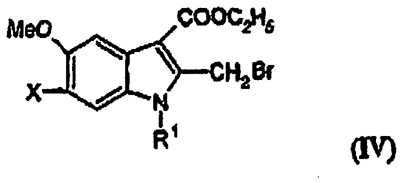 wobei X und R¹ wie oben definiert sind, mit dem entsprechenden nukleophilen Reagenz, ausgewählt unter dem entsprechenden Phenol, Thiophenol und einem das Fragment N(R)₂ umfassenden sekundären Amin zur Herstellung der Verbindungen der Formel V 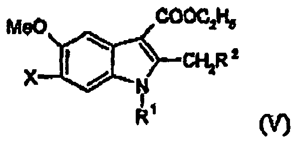 wobei X, R¹ und R² wie oben definiert sind, umgesetzt wird, wobei die Estergruppe zur Herstellung der Säure der allgemeinen Formel VI 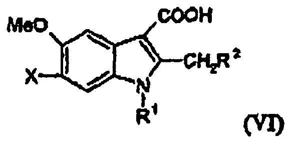 verseift wird, wobei X, R¹ und R² wie oben definiert sind, und die erhaltene Säure der Formel VI unmittelbar danach oder nach Umwandlung in das entsprechende Säurehalogenid mit dem entsprechenden, das Fragment N(R)₂ umfassenden sekundären Amin zur Herstellung der Verbindung der Formel I in freier Form oder in Form des pharmazeutisch verträglichen Salzes umgesetzt wird.

3. Verfahren nach Anspruch 2, **dadurch gekennzeichnet, dass** die Verbindung der allgemeinen Formel IV, hergestellt durch Methylierung der Verbindung der Formel II, verwendet wird, 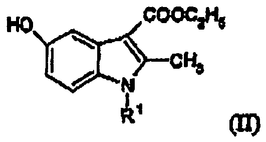 wobei X Wasserstoff bedeutet, R¹ wie oben definiert ist, zur Herstellung des entsprechenden 5-Methoxyindol-3-ylcarbonsäureesters der Formel III 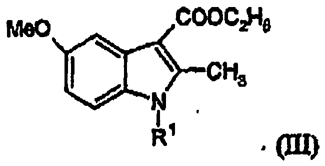 wobei R¹ wie oben definiert ist, unter nachfolgender Bromierung desselben.

4. Verfahren nach einem der Ansprüche 2 und 3, **dadurch gekennzeichnet, dass** die Säure der Formel VI 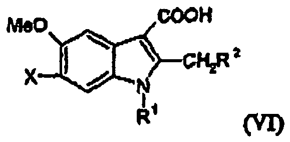 worin R¹ und R² wie oben definiert sind, mit Thionylchlorid zum entsprechenden Säurechlorid der Formel VII umgesetzt wird, 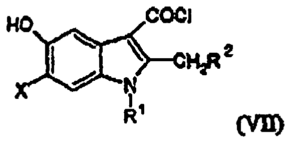 worin R¹ und R² wie oben definiert sind, das ohne Abtrennung oder nach Abtrennung aus dem Reaktionsgemisch mit dem das Fragment N(R)₂ umfassenden sekundären Amin umgesetzt wird.

5. Verfahren nach Anspruch 4, **dadurch gekennzeichnet, dass** die Verbindung der Formel VI, hergestellt durch Verseifung der Verbindung der Formel V, verwendet wird, 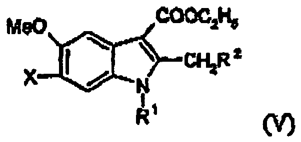 worin X, R¹ und R² wie oben definiert sind.

6. Verbindung der allgemeinen Formel I zur Herstellung eines Medikaments zur Behandlung von Influenza A 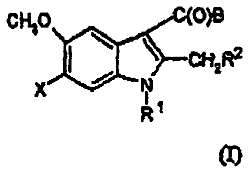 worin X, R¹, R² und B wie in Anspruch 1 definiert sind.

## Revendications

1. Composés de la formule générale (I) 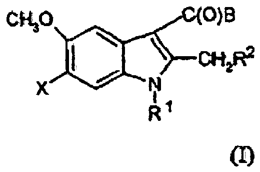 dans laquelle B est un groupe -N(R)₂,
chaque R est indépendamment sélectionné parmi un groupe alkyle en C₁ à C₄ et peut être identique ou différent, ou les deux groupes R conjointement à un atome d'azote auquel ils sont liés, forment un cycle hétérocyclique à 5 à 6 chaînons contenant 1 à 2 hétéroatome(s) choisi(s) parmi l'azote, l'oxygène et le soufre, tel que la pyrrolidine, la pipéridine, la pipérazine ou la morpholine, auquel chacun des cycles hétérocycliques ci-dessus mentionnés peut être substitué par un groupe alkyle en C₁ à C₄, phényle, benzyle, phénéthyle, carbonylamino, alkyle-COOC₁₋₄ ou alkyle-COOC₁₋₄ et phényle, qui peut également être substitué et qui ont des substituants sélectionnés parmi un atome d'halogène, un groupe alkyle en C₁ à C₄, alcoxy en C₁ à C₄, et le groupe alkyle dans lesdits groupes peut être linéaire ou ramifié ;
R¹ est un groupe alkyle en C₁ à C₄, phényle, éventuellement substitué par un groupe alkyle en C₁ à C₄ ou alcoxy en C₁ à C₄, des atomes d'halogène ; R² est un groupe alkyle en C₁ à C₄, -S-phényle, -S-benzyle, -O-phényle, dans lequel dans chacun des groupes ci-dessus mentionnés le cycle phénylique est éventuellement substitué par un groupe alkyle en C₁ à C₄, alcoxy en C₁ à C₄, des atomes d'halogène, ou R² est un groupe -N(R)₂ ;
X est un atome d'hydrogène ou un atome d'halogène sélectionné parmi Br, Cl, I et leurs sels pharmaceutiquement acceptables.

2. Procédé de préparation des composés de la formule générale (I) 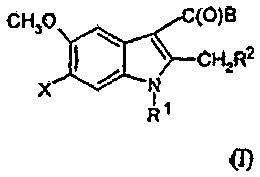 dans laquelle B, X, R¹ sont tels que définis selon la revendication 1, R² est N(R)₂, qui est **caractérisé en ce que** le composé de la formule (IV) 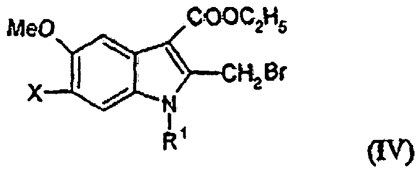 dans laquelle X, R¹ sont tels que définis ci-dessus,
est mis à réagir avec le réactif nucléophile correspondant, sélectionné parmi le phénol correspondant, le thiophénol, l'amine secondaire incluant le fragment N(R)₂, pour la préparation du composé de la formule générale (V) 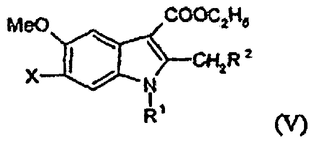 dans laquelle X, R¹, R² sont tels que définis ci-dessus,
dans laquelle le groupe ester alors est saponifié avec la préparation de l'acide de la formule générale (VI) 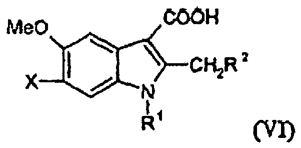 dans laquelle X, R¹, R² sont tels que définis ci-dessus,
et l'acide obtenu de la formule (VI) immédiatement, ou après la transformation dans l'halogénure d'acide correspondant, est mis à réagir avec l'amine secondaire correspondante incluant le fragment N(R)₂, avec la préparation du composé de la formule I sous une forme libre ou sous la forme du sel pharmaceutiquement acceptable.

3. Procédé selon la revendication 2, qui est **caractérisé en ce que** le composé de la formule générale (IV) préparé par la méthylation du composé de la formule (II), est utilisé 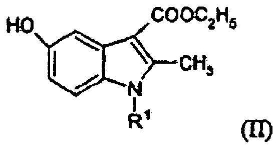 dans laquelle X est un atome d'hydrogène, R¹ est tel que défini ci-dessus, avec la préparation de l'ester d'acide 5-méthoxyindol-3-yl carboxylique correspondant de la formule (III) 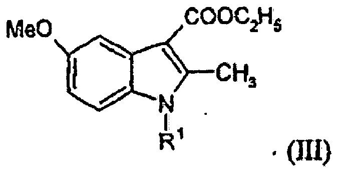 dans laquelle R¹ est tel que défini ci-dessus,
et avec la bromation suivante de celui-ci.

4. Procédé selon l'une quelconque des revendications 2 et 3, qui est **caractérisé en ce que** l'acide de la formule VI 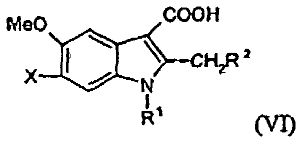 dans laquelle R¹, R² sont tels que définis ci-dessus,
est mis à réagir avec le chlorure de thionyle pour l'obtention du chlorure d'acide correspondant de la formule (VII) 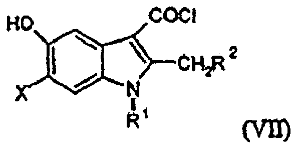 dans laquelle R¹, R² sont tels que définis ci-dessus,
qui est mis à réagir, sans séparation ou après la séparation de la masse réactionnelle, avec l'amine secondaire incluant le fragment N(R)₂.

5. Procédé selon la revendication 4, qui est **caractérisé en ce que** le composé de la formule VI préparé par saponification du composé de la formule (V), est utilisé 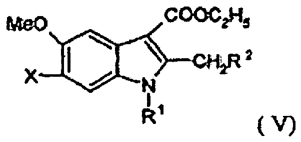 dans laquelle X, R¹, R² sont tels que définis ci-dessus.

6. Composé de la formule générale I pour la préparation d'un médicament pour le traitement de la grippe A 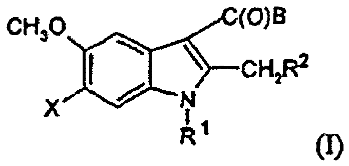 dans laquelle X, R¹, R², B sont tels que définis selon la revendication 1.
